# EUROPEAN PATENT APPLICATION

(11) **EP 4 821 587 A2**
(43) Date of publication of application: **14.10.2026**
(21) Application number: 26174889.1
(22) Date of filing: 29.03.2019
(51) Int. Cl.: B01F 23/40

(54) **METHODS FOR CONTINUOUS MANUFACTURE OF LIPOSOMAL DRUG PRODUCTS**

(30) Priority: 30.03.2018 US 201862650372 P
(62) Document number(s) of the earlier application(s) in accordance with Art. 76 EPC: 19774338.8 / 3 773 505
(71) Applicant: Insmed Incorporated, Bridgewater, NJ 08807-1704 (US)
(72) Inventor: WORSHAM, Robert, Bridgewater, 08807-1704 (US)
(74) Representative: Cooley (UK) LLP

(57) **Abstract**

Provided herein are methods for making liposomal API formulations via continuous in-line diafiltration processes. Also provided herein are liposomal API formulations manufactured by the disclosed methods.

## Description

### CROSS REFERENCE TO RELATED APPLICATION

This application claims priority from U.S. Provisional Application Serial No. 62/650,372, filed March 30, 2018, the disclosure of which is incorporated by reference herein in its entirety for all purposes.

### BACKGROUND OF THE INVENTION

Continuous manufacturing is a process whereby raw materials constantly flow into a process and intermediates or final product constantly flow out. Such processing has been employed in non-pharmaceutical industries and has recently been adopted in some types of pharmaceutical processes such as the synthesis of active pharmaceutical ingredients (APIs) and generation of solid oral dosage forms (tablets, etc.) (Kleinebudde et al. (Eds.), Continuous Manufacturing of Pharmaceuticals, Wiley-VCH, Hoboken 2017; Subramanian, G. (Ed.), Continuous Process in Pharmaceutical Manufacturing, Wiley-VCH, Weinheim 2015).

In recent history, continuous manufacturing has been used for the production of biologics. The manufacture of biologics has continued to develop the requirements and aspects to consider surrounding unit operations such as cell culture, chromatography, viral inactivation and various methods for tangential flow filtration (TFF), such as alternating tangential filtration (ATF) and single pass tangential flow filtration (SPTFF) (Subramanian, G. (Ed.), Continuous Process in Pharmaceutical Manufacturing, Wiley-VCH, Weinheim 2015)). ATF, for example, is a means of performing buffer/medium exchange with lower shear forces as compared to TFF. Continuous perfusive cell culture has used ATF to support continuous medium exchange with highly concentration suspensions (Castilho, Continuous Animal Cell Perfusion Processes: The First Step Toward Integrated Continuous Manufacturing, in: Subramanian, G. (Ed.), Continuous Process in Pharmaceutical Manufacturing, Wiley-VCH, Weinheim 2015, pp. 115-153; Whitford, Single-Use Systems Support Continuous Bioprocessing by Perfusion Culture, in: Subramanian, G. (Ed.), Continuous Process in Pharmaceutical Manufacturing, Wiley-VCH, Weinheim 2015, pp. 183-226).

Single pass tangential flow filtration (SPTFF) has been evaluated as well for concentrating protein, allowing this process step to happen in a continuous fashion instead of the batch mode required by traditional TFF (Brower et al. Monoclonal Antibody Continuous Processing Enabled by Single Use, in: Subramanian, G. (Ed.), Continuous Process in Pharmaceutical Manufacturing, Wiley-VCH, Weinheim 2015, pp. 255-296; Jungbauer, Continuous downstream processing of biopharmaceuticals. Trends in Biotechnolgy. 2013, 8, 479-492; Dizon-Maspat et al., Single pass tangential flow filtration to debottleneck downstream processing for therapeutic antibody production. Biotechnol Bioeng. 2012, 4, 962-70).

Other aspects for commercial implementation of continuous manufacturing such as a process analytical technology (PAT) requirement and use of single-use or disposable componentry have been explored. The implementation of single-use or disposable technology provides the same conceptual benefits as it would for a batch process, but increased in magnitude as more product is generated per single-use/disposable item.

The present invention addresses the need for a continuous manufacturing process for liposomal active pharmaceutical ingredients (liposomal APIs), such as liposomal drug products.

### SUMMARY OF THE INVENTION

In one aspect of the invention, a method for manufacturing a liposomal API formulation in a continuous manner is provided.

One embodiment of the method for manufacturing the liposomal API formulation comprises mixing a lipid solution comprising a lipid dissolved in an organic solvent with an aqueous API solution, wherein the lipid solution and aqueous API solution are mixed from two separate streams in an in-line fashion, and wherein a liposomal encapsulated API is formed at the intersection of the two streams. The method further comprises introducing the liposomal encapsulated API into a central vessel comprising a first inlet, a second inlet, a first outlet and a second outlet, through the first inlet. The first outlet of the central vessel is in fluid communication with an inlet of a first tangential flow filtration (TFF) unit. The first TFF unit comprises the aforementioned inlet and a first and second outlet. The first outlet of the first TFF unit is in fluid communication with the second inlet of the central vessel and the second outlet of the first TFF unit is a waste outlet. The second outlet of the central vessel is in fluid communication with an inlet of a second TFF unit comprising the inlet and a first and second outlet. The first outlet of the second TFF unit is a retentate outlet and the second outlet of the second TFF unit is a waste (permeate) outlet. The method further comprises continuously flowing the liposomal encapsulated API into the first TFF unit for a first period of time. The liposomal encapsulated API enters the first TFF unit through the TFF inlet and exits through the first outlet. The method further comprises flowing the liposomal encapsulated API from the central vessel through the inlet of the second TFF unit for a second period of time and collecting the liposomal API formulation from the first outlet of the second TFF unit.

In one embodiment, the method comprises flowing the liposomal encapsulated API from the central vessel into one or more additional TFF units prior to flowing the liposomal API formulation into the second TFF unit.

In one embodiment, the second TFF unit is a single pass TFF unit (SPTFF).

In a second embodiment, the method for manufacturing the liposomal API formulation comprises mixing a lipid solution comprising a lipid dissolved in an organic solvent with an aqueous API solution, wherein the lipid solution and aqueous API solution are mixed from two separate streams in an in-line fashion, and wherein a liposomal encapsulated API is formed at the intersection of the two streams. The method further comprises introducing the liposomal encapsulated API into a central vessel comprising an inlet and an outlet, through the inlet. The outlet is in fluid communication with an inlet of a first tangential flow filtration (TFF) unit. The first TFF unit comprises the aforementioned inlet and a first and second outlet. The first outlet of the first TFF unit is in fluid communication with the inlet of a second TFF and the second outlet of the first TFF unit is a waste (permeate) outlet. The second TFF comprises the aforementioned inlet and a first and second outlet. The first outlet of the second TFF unit is a retentate outlet and the second outlet of the second TFF unit is a waste (permeate) outlet. The method further comprises continuously flowing the liposomal encapsulated API into the first TFF unit for a first period of time. The liposomal encapsulated API enters the first TFF unit through the TFF inlet and exits through the first outlet. The method further comprises flowing the liposomal encapsulated API from the first outlet of the first TFF through the inlet of the second TFF unit for a second period of time and collecting the liposomal API formulation from the first outlet of the second TFF unit.

In a further embodiment, the method comprises flowing the liposomal encapsulated API from the central vessel into one or more additional TFF units prior to flowing the liposomal API formulation into the second TFF unit.

In one embodiment, the second TFF unit is a single pass TFF unit (SPTFF).

In a third embodiment, the method for manufacturing a liposomal API formulation comprises mixing a lipid solution comprising a lipid dissolved in an organic solvent with an aqueous API solution, wherein the lipid solution and aqueous API solution are mixed from two separate streams in an in-line fashion, and wherein liposomal encapsulated API is formed at the intersection of the two streams. The method further comprises introducing the liposomal encapsulated API into a central vessel comprising a first inlet, a second inlet, a first outlet and a second outlet, through the first inlet. The first outlet is in fluid communication with an inlet of a first tangential flow filtration (TFF) unit comprising the inlet and a first and second outlet. The first outlet of the first TFF unit is in fluid communication with the second inlet of the first central vessel and the second outlet of the first TFF unit is a waste outlet. The second outlet of the first central vessel is in fluid communication with a first inlet of a second central vessel. The second central vessel comprises the first inlet, a second inlet, a first outlet and a second outlet, and the first outlet of the second central vessel is in fluid communication with an inlet of a second tangential flow filtration (TFF) unit comprising the inlet and a first and second outlet. The first outlet of the second TFF unit is in fluid communication with the second inlet of the second central vessel, the second outlet of the second TFF unit is a waste outlet. The second outlet of the second central vessel is in fluid communication with an inlet of a third TFF unit comprising the inlet and a first and second outlet, the first outlet of the third TFF unit is a retentate outlet and the second outlet of the third TFF unit is a waste (permeate) outlet. The method further comprises continuously flowing the liposomal encapsulated API into the first TFF unit for a first period of time, wherein the liposomal encapsulated API enters the first TFF unit through the TFF inlet and exits through the first outlet. The method further comprises flowing the liposomal encapsulated API from the first central vessel into the second central vessel for a second period of time and continuously flowing the liposomal encapsulated API into the second TFF unit from the second central vessel for a third period of time. The liposomal encapsulated API enters the second TFF unit through the TFF inlet and exits through the first outlet. The method further comprises flowing the liposomal encapsulated API from the second central vessel through the inlet of the third TFF unit for a fourth period of time; and collecting the liposomal encapsulated API formulation from the first outlet of the third TFF unit.

In one aspect of the third embodiment, the method comprises flowing the liposomal encapsulated API from the second central vessel into one or more additional TFF units prior to flowing the liposomal API formulation into the third TFF unit.

In another aspect of the third embodiment, the third TFF unit is a single pass TFF unit (SPTFF).

In one embodiment of the methods provided herein, mixing the lipid solution and the aqueous API solution results in the formation of a API coacervate. In a further embodiment, the API coacervate initiates lipid bilayer formation around the API coacervate.

In one embodiment of the methods provided herein, the API is an aminoglycoside. In a further embodiment, the aminoglycoside is amikacin, or a pharmaceutically acceptable salt thereof. In even a further embodiment, the amikacin is amikacin sulfate.

In one embodiment of the methods provided herein, a buffer is introduced into the first central vessel through a third inlet prior to the first period of time or during the first period of time.

In another aspect of the invention, a liposomal API formulation made by a continuous method described herein, is provided.

### BRIEF DESCRIPTION OF THE FIGURES

**Figure 1** is a liposomal API manufacturing process flow diagram. Ethanol/ether injection batch design method: lipid/solvent solution is directly fed into the central vessel. Formulations are refined in multi-step buffer exchange diafiltration and concentration steps.
**Figure 2** is a liposomal API manufacturing process flow diagram. Crossflow method: solvent/anti-solvent mix in-line at an intersection point. Formulations are refined in multi-step buffer exchange diafiltration and concentration steps.
**Figure 3** is a process design for continuous liposome API manufacturing. Single tank buffer exchange tangential flow filtration (TFF) and single stage concurrent concentrating single-pass tangential flow filtration (SPTFF).
**Figure 4** is a process design for continuous liposome API manufacturing. Continuous multistage (multi-vessel) buffer exchange TFF and single stage concurrent concentrating SPTFF.
**Figure 5** is a process design for continuous liposome API manufacturing. Single tank buffer exchange TFF and multistage concurrent concentrating SPTFF.
**Figure 6** is a process design for continuous liposome API manufacturing. Multistage buffer exchange (in-line diafiltration (ILDF)) with concurrent concentrating SPTFF.
**Figure 7** compares batch vs. continuous processing steps/times for a liposomal API product.

### DETAILED DESCRIPTION OF THE INVENTION

The present invention, in one aspect, relates to the use of continuous manufacturing processes for the manufacture of liposomal API products. The potential benefits of implementing a continuous manufacturing process without wishing to be bound by theory, include economic advantages (lower capital expenditures, smaller facility footprint, lower overall cost of goods sold (COGS)), as well as improved consistency and quality of product.

In another aspect, a liposomal API formulation manufactured by a process provided herein is provided.

One aspect of the method for manufacturing the liposomal API formulation provided herein comprises an initial liposomal API encapsulation step. The liposomal API encapsulation, in one embodiment, comprises mixing a lipid solution comprising a lipid dissolved in an organic solvent with an aqueous API solution, wherein the lipid solution and aqueous API solution are mixed from two separate streams in an in-line fashion, and wherein a liposomal encapsulated API is formed at the intersection of the two streams. In another embodiment, the liposomal API encapsulation takes place in a central vessel via an alcohol injection method.

The method, in a first embodiment, comprises introducing a liposomal encapsulated API into a central vessel or forming a liposomal encapsulated API in the central vessel. The central vessel comprises a first inlet, a second inlet, a first outlet and a second outlet. The liposomal encapsulated API in one embodiment, is introduced through the first inlet of the central vessel.

The first outlet of the central vessel is in fluid communication with an inlet of a first tangential flow filtration (TFF) unit.

The terms "tangential flow filtration unit" or "TFF unit" are art-known and mean a device that includes at least one housing (such as a cylinder or cartridge) and at least one cross-flow (tangential) filter positioned in the housing such that a large portion of the filter's surface is positioned parallel to the flow of a fluid (e.g., a liposomal suspension) through the unit. In one embodiment, a TFF unit includes one filter. In another embodiment, a TFF unit includes two filters. In yet another embodiment, the TFF unit includes three filters. TFF units are well-known in the art and are commercially available, e.g., from Pall Life Sciences. The housing can include a first inlet/outlet and a second inlet/outlet positioned, e.g., to allow fluid to pass through the first inlet/outlet, cross the at least one cross-flow filter, and through the second inlet/outlet. In some examples, a circuit system can include multiple TFF units, e.g., connected in series and/or in parallel. In the methods provided herein, TFF units can be connected in series and/or parallel to provide a fluid path of desired length. For example, 4, 5, 6, 7, 8, 9 or 10 TFF units can be connected in parallel and/or series in the methods provided herein. In one embodiment, 6, 7, 8, 9, 10, 11, 12, 13, 14 or 15 TFF units are connected in parallel and/or series in the methods provided herein. In another embodiment, from about 5 to about 20 or from about 5 to about 15 TFF units are connected in series in one of the methods provided herein.

In one embodiment, a circuit system that includes two or more TFF units can include fluid conduits fluidly connecting neighboring pairs of TFF units in the system. In other examples, a circuit system can include two or more TFF units fluidly connected by fluid conduits. The TFF unit, in one embodiment, is a single pass TFF (SPTFF) unit. In another embodiment, the two or more TFF units comprise a TFF unit and a SPTFF unit.

The first TFF unit comprises the aforementioned inlet and a first and second outlet. The first outlet of the first TFF unit is the retentate outlet, and is in fluid communication with the second inlet of the central vessel and the second outlet of the first TFF unit is a waste (permeate) outlet. The second outlet of the central vessel is in fluid communication with an inlet of a second TFF unit comprising the inlet and a first and second outlet. The first outlet of the second TFF unit is a retentate outlet and the second outlet of the second TFF unit is a waste (permeate) outlet.

The method further comprises continuously flowing the liposomal encapsulated API into the first TFF unit for a first period of time. The liposomal encapsulated API enters the first TFF unit through the TFF inlet and exits through the first outlet. The method further comprises flowing the liposomal encapsulated API from the central vessel through the inlet of the second TFF unit for a second period of time and collecting the liposomal API formulation from the first outlet of the second TFF unit.

"Fluid communication" as used herein, means direct or indirect fluid communication, e.g., directly through a connection port or indirectly through a process unit such as a TFF unit, central vessel, etc.

In one embodiment, the method comprises flowing the liposomal encapsulated API from the central vessel into one or more additional TFF units prior to flowing the liposomal API formulation into the second TFF unit.

In one embodiment, the second TFF unit is a single pass TFF unit (SPTFF).

The method, in a second embodiment, comprises introducing a liposomal encapsulated API into a central vessel or forming a liposomal encapsulated API in the central vessel. The central vessel comprises an inlet and an outlet. The liposomal encapsulated API in one embodiment, is introduced through the inlet of the central vessel.

The outlet of the central vessel is in fluid communication with an inlet of a first tangential flow filtration (TFF) unit. The first TFF unit comprises the aforementioned inlet and a first and second outlet. The first outlet of the first TFF unit is in fluid communication with the inlet of a second TFF unit comprising the inlet and a first and second outlet The first outlet of the second TFF unit is a retentate outlet and the second outlet of the second TFF unit is a waste (permeate) outlet.

The method further comprises continuously flowing the liposomal encapsulated API into the first TFF unit for a first period of time. The liposomal encapsulated API enters the first TFF unit through the TFF inlet and exits through the first outlet. The method further comprises flowing the liposomal encapsulated API from the first outlet of the first TFF through the inlet of the second TFF unit for a second period of time and collecting the liposomal API formulation from the first outlet of the second TFF unit.

In one embodiment, the method comprises flowing the liposomal encapsulated API from the central vessel into one or more additional TFF units prior to flowing the liposomal API formulation into the second TFF unit.

In one embodiment, the second TFF unit is a single pass TFF unit (SPTFF).

In a third embodiment of a continuous liposomal API formulation manufacturing method, the method comprises introducing the liposomal encapsulated API into a first central vessel or forming the liposomal encapsulated API in the first central vessel. The first central vessel comprises a first inlet, a second inlet, a first outlet and a second outlet. The liposomal encapsulated API in one embodiment is introduced into the central vessel through the first inlet. The first outlet of the first central vessel is in fluid communication with an inlet of a first tangential flow filtration (TFF) unit comprising the inlet and a first and second outlet. The first outlet of the first TFF unit is in fluid communication with the second inlet of the first central vessel and the second outlet of the first TFF unit is a waste (permeate) outlet. The second outlet of the first central vessel is in fluid communication with a first inlet of a second central vessel.

The second central vessel comprises the first inlet, a second inlet, a first outlet and a second outlet. The first outlet of the second central vessel is in fluid communication with an inlet of a second tangential flow filtration (TFF) unit comprising the inlet and a first and second outlet. The first outlet (retentate outlet) of the second TFF unit is in fluid communication with the second inlet of the second central vessel, the second outlet of the second TFF unit is a waste (permeate) outlet. The second outlet of the second central vessel is in fluid communication with an inlet of a third TFF unit comprising the inlet and a first and second outlet. The first outlet of the third TFF unit is a retentate outlet and the second outlet of the third TFF unit is a waste (permeate) outlet.

In this embodiment, the method further comprises continuously flowing the liposomal encapsulated API into the first TFF unit for a first period of time, wherein the liposomal encapsulated API enters the first TFF unit through the TFF inlet and exits through the first outlet. The method further comprises flowing the liposomal encapsulated API from the first central vessel into the second central vessel for a second period of time and continuously flowing the liposomal encapsulated API into the second TFF unit from the second central vessel for a third period of time. The liposomal encapsulated API enters the second TFF unit through the TFF inlet and exits through the first outlet. The method further comprises flowing the liposomal encapsulated API from the second central vessel through the inlet of the third TFF unit for a fourth period of time; and collecting the liposomal encapsulated API formulation from the first outlet of the third TFF unit.

The "first period of time", "second period of time", "third period of time" and "fourth period of time" can each be selected by the user of the method, depending in part on the selection of materials used to formulate the liposomal API, and/or the desired concentration of the liposomal API formulation. In one embodiment, the first period of time", "second period of time", "third period of time" and/or "fourth period of time" are each independently 1 h, 2 h, 3 h, 4 h, 5 h, 6 h, 8 h, 12 h, 18 h, 24 h, 36 h, 48 h, 60 h, 72 h, 84 h, 96 h or 108 h.

In each of the methods provided herein, an initial liposome formation step is employed. A variety of liposomal encapsulation methods are available to those of ordinary skill in the art, and can be employed herein. The liposomal encapsulation step, in one embodiment, is carried out upstream of an initial filtration step. The liposomal encapsulation, in one embodiment, takes place in a first central vessel. In another embodiment, the liposomal encapsulation takes place upstream of the first central vessel, and is provided to the first central vessel.

Liposomes were first discovered the early-1960s and a number of strategies have been demonstrated for their manufacture since (Mozafari. Liposomes: an overview of manufacturing techniques. Cell Mol Biol Lett. 2005, 10(4), 711-719; Maherani et al., Liposomes: A Review of Manufacturing Techniques and Targeting Strategies. Current Nanoscience. 2011, 7(3), 436-445; each of which is incorporated by reference herein in its entirety for all purposes).

Frequently, liposomal products are reformulations of compendial APIs meant to alleviate adverse clinical side effects and/or provide a more targeted delivery as compared to systemic dosages (Maurer et al. Expert Opinion on Biological Therapy. 2001, 6, 923-947; Lian and Ho. Expert J Pharm Sci. 2001, 6, 667-680; each of which is incorporated by reference herein in its entirety for all purposes).

However, until recently, the application of liposomal products in pharmaceutical development has suffered from a lack of reliable manufacturing methods with sufficient throughput to enable commercial scale-up. Table 1 provides a summary of various liposome formation methods. In embodiments described herein, a liposomal API can be provided to the first central vessel or in the first central vessel via a supercritical fluid method, dense gas method, alcohol injection or crossflow method.

| **Table 1.** Liposome formation methods | | |
|---|---|---|
| **Method** | **Mechanism** | **Reference** |
| Bangham | Rehydration of thin lipid film | Bangham et al., The action of steroids and streptolysin S on the permeability of phospholipid structures to cations. J. Mol Biol. 1965, 13, 253-259. |
| | | Bangham et al., Diffusion of univalent ions across the lamellae of swollen phospholipids. J. Mol Biol. 1965, 13, 238-252. |
| | | Deamerand Bangbam, Large volume liposomes by an ether vaporization method. Biochimica et Biophysica Acta. 1976, 443, 629-634. |
| Sonication method | Sonication of an aqueous lipid suspension | Perrett et al. A simple method for the preparation of liposomes for pharmaceutical applications: characterization of the liposomes. J Pharm Pharmacol. 1991, 43(3), 154-161. |
| Reverse phase evaporation | Aqueous phase added to organic phase and evaporated to form liposomes | Mcure et al., Conventional and dense gas technology for the production of liposomes: A review. AAPS Pharma. Sci. Tech. 2008, 9(3), 798-809. |
| | | Szoka Jr. and Papahadjopoulos. Procedure for preparation of liposomes with large internal aqueous space and high capture by reverse-phase evaporation. Proc. Natl. Acad. Sci., USA. 1978, 75(9), 4194-4198. |
| Detergent depletion | Liposomes formed through detergent lipid interaction | Brunner et al., Single bilayer vesicles prepared without sonication. Physico-chemical properties. Biochim Biophys Acta. 1976, 455(2), 322-331. |
| | | Lasch et al., Preparaton of liposomes, in: Torchilin, V., Wessig, V. (Ed.), Liposomes: A practical approach, Oxford University Press, New York, 2003, p 3-29. |
| Microfluidic channel | Intersection of lipid/API solutions in micro-channcls | Jahn et al., Microfluidic directed formation of liposomes of controlled size. Langmuir. 2007, 23(11), 6289-6293. |
| High pressure homogenization | Liposome formation through high pressure mixing | Barnadas-Rodriguez and Sabes, Factors involved in the production of liposomes with a high-pressure homogenizer. Int. J. Pharma. 2001, 213, 175-186. |
| | | Carugo et al., Liposome production by microfluidics: potential and limiting factors. Scientific Reports. 2016, 6, DOI:10.1038/srep25876. |
| Heating method | Heating of a lipid aqueous/glycerol solution to form liposomes | Mozafari. Liposomes: an overview of manufacturing techniques. Cell Mol Biol Lett. 2005, 10(4), 711-719. |
| | | Mortazavi et al. Preparation of liposomal gene therapy vectors by a scalable method without using volatile solvents or detergents. J. Biotechnol. 2007, 129(4), 604-613. Mozafari et al., Development of non-toxic liposomal formulations for gene and drug delivery to the lung. Technol. Health Care., 2002, 10(3-4), 342-344. |
| Supercritical fluid methods | Use of supercritical fluids as solvent for lipids instead of organic solvents | Meure et al. Conventional and dense gas technology for the production of liposomes: A review. AAPS Pharma. Sci. Tech. 2008, 9(3), 798-809. |
| | | Santo et al. Liposomes Size Engineering by Combination of Ethanol Injection and Supercritical Processing. J Pharm Sci. 2015, 104(11), 3842-3850. |
| | | Santo et al. Liposomes prepration using a supercritical fluid assisted continuous process. Chemical Engineering Journal. 2014, 249, 153-159. |
| | | Campardelli et al., Efficient encapsulation of proteins in submicro liposomes using a supercritical fluid assisted continuous process. The Journal of Supercritical Fluids. 2016, 107, 163-169. |
| | | Frederiksen et al. Preparation of Liposomes Encapsulating Water-Soluble Compounds Using Supercritical Carbon Dioxide. Journal of Pharmaceutical Sciences. 1997, 86(8), 921-928. |
| | | Otake et al., Development of a new preparation method of liposomes using supercritical carbon dioxide. Langmuir. 2001, 17(13), 3898-3901. |
| Dense Gas methods | Use of dense gas as solvent for lipids instead of organic solvents | Meure et al., Conventional and dense gas technology for the production of liposomes: A review. AAPS Pharma. Sci. Tech. 2008, 9(3), 798-809. |
| | | Otake et al., Development of a new preparation method of liposomes using supercritical carbon dioxide. Langmuir. 2001, 17(13), 3898-3901. |
| | | Anton et al., Preparation of a liposome dispersion containing an active agent by compression-decompression. EP616801, 1994. |
| Ethanol/ether injection | Precipitation of liposome from organic phase into aqueous | Jaafar-Maalej et al. Ethanol injection method for hydrophilic and lipophilic drug-loaded liposome preparation. Journal of Liposome Research. 2010, 20:3, 228-243. |
| | | Santo et al. Liposomes Size Engineering by Combination of Ethanol Injection and Supercritical Processing. J Pharm Sci. 2015, 104(11), 3842-3850. Batzri and Korn. Single bilayer vesicles prepared without sonication. Biochim Biophys Acta. 1973, 298, 1015-1019. Deamer and Bangham. Large volume liposomes by an ether vaporization method. Biochim Biophys Acta-Biomembr. 1976, 443(3), 629-634. |
| Crossflow method | In-line Precipitation of liposome from organic phase into aqueous | Wagner et al. GMP Production of Liposomes - A New Industrial Approach. Journal of Liposome Research. 2006, 16:3, 311-319. |
| | | Wagner et al. Liposomes produced in a pilot scale: production, purification and efficiency aspects. European Journal of Pharmaceutics and Biopharmaceutics. 2002, 54. 213-219. |
| | | Wagner et al. The crossflow injection technique: An improvement of the ethanol injection method. Journal of Liposome Research, 2002, 12:3, 259-270. |
| | | Wagner and Vorauer-Uhl. Liposome Technology for Industrial Purposes. Journal of Drug Delivery. 2011, 2011, DOI: 10.1155/2011/591325. |
| | | Wagner et al. Enhanced protein loading into liposomes by the multiple crossflow injection technique. Journal of Liposome Research. 2002, 12:3, 271-283. |

Generally, strategies for liposome synthesis focus on addressing and optimizing one or several of the key driving forces of vesicle assembly including the component solubilities, concentrations, and process thermodynamic parameters (e.g., temperature, pressure, etc.) (Mozafari (2005). Cell Mol Biol Lett., 10(4), pp. 711-719; Maherani et al.

(2011). Current Nanoscience. 7(3), pp. 436-445, each of which is incorporated by reference herein in its entirety for all purposes). Manufacture methods can be designed to fine-tune liposomes with various properties and, in doing so, can lend both advantages and disadvantages amenable to large-scale processing. In addition, selection of the manufacturing method often depends on the end product requirements for clinically efficacy including liposome size and size distribution, lipid composition, and the API release characteristics, together, which dictate the pharmacokinetic demonstration of adsorption, distribution, metabolism, and elimination (ADME).

The earliest methods for liposome formation began with multistep synthetic strategies involving the rehydration of thin phospholipid films in aqueous media which resulted in the spontaneous formation of lipid structures of varying sizes, shapes, and lamella (Bangham et al. The action of steroids and streptolysin S on the permeability of phospholipid structures to cations. J. Mol. Biol. 1965, 13, 253-259; Bangham et al. Diffusion of univalent ions across the lamellae of swollen phospholipids. J. Mol. Biol. 1965, 13, 238-252; Deamer and Bangham. Large volume liposomes by an ether vaporization method. Biochimica et Biophysica Acta. 1976, 443, 629-634.). For uniform product generation, these suspensions required post-formation mechanical size manipulations strategies (Bamadas-Rodriguez and Sabes. Factors involved in the production of liposomes with a high-pressure homogenizer. Int. J. Pharma. 2001, 213, 175-186; Carugo ct al. Liposome production by microfluidics: potential and limiting factors. Scientific Reports. 2016, 6, DOI:10.1038/srep25876). More recently, efforts have been dedicated towards investigating the possibility for single-step scalable techniques that involve programmable online flow-based strategies to arrive at the controlled precipitation and subsequent self-assembly of phospholipids into uniform structures, which can be implemented in a regulated pharmaceutical environment (Wagner et al. Production of Liposomes - A New Industrial Approach. Journal of Liposome Research. 2006, 16:3, 311-319.).

In one embodiment, an alcohol injection or crossflow technique is employed in one of the manufacturing methods provided herein. The liposomes are formed in the first central vessel, e.g., via alcohol injection, or provided to the first central vessel after liposome formulation at an upstream in-line formation step. In one embodiment, one of the liposome formation methods set forth in International patent application publication nos. WO 2007/117550 (crossflow); WO 2007/011940 (crossflow) and/or WO 2004/110346 (alcohol injection), each of which is incorporated by reference herein in its entirety for all purposes, is employed herein in an initial liposome formation step.

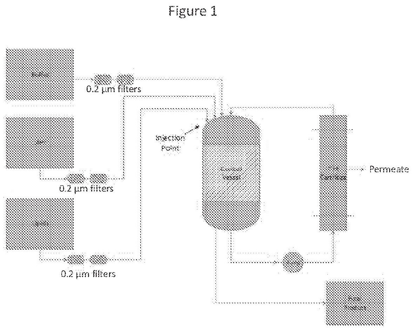

In alcohol injection and/or crossflow liposomal formation embodiments, dissolved lipids are precipitated from an organic solvent into an aqueous solution (antisolvent) by means of reciprocal diffusion of the alcohol and aqueous phases (Figures 1-2) (Jaafar-Maalej et al. Ethanol injection method for hydrophilic and lipophilic drug-loaded liposome preparation. Journal of Liposome Research. 2010, 20:3, 228-243; Wagner et al. Liposomes produced in a pilot scale: production, purification and efficiency aspects. European Journal of Pharmaceutics and Biopharmaceutics. 2002, 54, 213-219; Wagner et al. The crossflow injection technique: An improvement of the ethanol injection method. Journal of Liposome Research. 2002, 12:3, 259-270; Wagner and Vorauer-Uhl. Liposome Technology for Industrial Purposes. Journal of Drug Delivery. 2011, 2011, DOI: 10.1155/2011/591325; Wagner et al. Enhanced protein loading into liposomes by the multiple crossflow injection technique. Journal of Liposome Research. 2002, 12:3, 271-283). A change in the local solubility of the lipids during this process ultimately leads to the spontaneous formation of liposomes that encapsulate a small volume of the aqueous solution. Depending on the chemical nature of the API, it can be encapsulated in the aqueous core or embedded in the lipid bilayer of the liposome. Parameters for the formation of liposomes by this method are residence time and geometry of the mixing/intersection of organic-solvated lipid and the antisolvent, which are dictated by programmed flow conditions. After liposome formation, the mixture containing undesired organic solvent and unencapsulated API can then be refined to the desired formulation strength and composition using TFF or similar methods, as set forth herein.

It should be noted that the supercritical fluid and dense gas methods use their namesakes as the solvent for the lipid solution while the injection and crossflow method use organic solvents. Without wishing to be bound by theory, it is thought that supercritical and dense gas feed solutions require high pressure that would be difficult adapt to a continuous design (Mcurc ct al. Conventional and dense gas technology for the production of liposomes: A review. AAPS Pharma. Sci. Tech. 2008, 9(3), 798-809; Santo et al. Liposomes Size Engineering by Combination of Ethanol Injection and Supercritical Processing. J Pharm Sci. 2015, 104(11), 3842-3850; Santo et al. Liposomes preparation using a supercritical fluid assisted continuous process. Chemical Engineering Journal. 2014, 249, 153-159; Campardelli et al. Efficient encapsulation of proteins in submicro liposomes using a supercritical fluid assisted continuous process. The Journal of Supercritical Fluids. 2016, 107, 163-169: Frederiksen et al. Preparation of Liposomes Encapsulating Water-Soluble Compounds Using Supercritical Carbon Dioxide. Journal of Pharmaceutical Sciences. 1997, 86(8), 921-928; Otake et al. Development of a new preparation method of liposomes using supercritical carbon dioxide. Langmuir. 2001, 17(13), 3898-3901; Anton et al. Preparation of a liposome dispersion containing an active agent by compression-decompression. EP616801, 1994). With continuous formulation of the feed solutions, the liposome formation step can proceed indefinitely. By adding continuous steps, continuous manufacturing of liposomal API products can be carried out.

In one aspect, the present invention provides a method for continuous manufacture of a liposomal product comprising an active pharmaceutical ingredient (API) encapsulated by a liposome, or complexed with a liposome. In some embodiments, the API is an aminoglycoside. In a further embodiment, the aminoglycoside is amikacin, or a pharmaceutically acceptable sat thereof.

A "pharmaceutically acceptable salt" includes both acid and base addition salts. A pharmaceutically acceptable addition salt refers to those salts which retain the biological effectiveness and properties of the free bases, which are not biologically or otherwise undesirable, and which are formed with inorganic acids such as, but are not limited to, hydrochloric acid (HCl), hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid (e.g., as lactate), lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, acetic acid (*e.g.,* as acetate), tartaric acid, thiocyanic acid, p-toluenesulfonic acid, trifluoroacetic acid (TFA), undecylenic acid, and the like. In one embodiment, the pharmaceutically acceptable salt is HCl, TFA, lactate or acetate.

A pharmaceutically acceptable base addition salt retains the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Inorganic salts include the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminocthanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine. N-ethylpiperidine, polyamine resins and the like. Organic bases that can be used to form a pharmaceutically acceptable salt include isopropylamine, diethylamine, ethanolamine, trimethylamine, dicyclohexylamine, choline and caffeine.

The term "unit operation" is a term of art and means a functional step that can be performed in a process of manufacturing a liposomal encapsulated API. For example, a unit of operation can be mixing a lipid and API to form a liposomal encapsulated API, filtering (e.g., removal of contaminant bacteria, removal of free API, removal of free lipid, etc., from a fluid containing a liposomal encapsulated API), adjusting the ionic concentration and/or pH of a fluid containing the liposomal encapsulated API, removing unwanted salts.

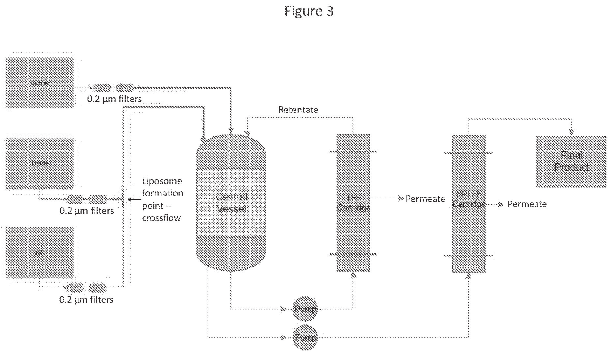

The unit operations downstream of liposome formation in the continuous manufacturing processes provided herein are used to refine the liposomal API formulation to the desired specification. Frequently, unit operations such as TFF are used to remove undesired elements, such as non-encapsulated API or organic solvent, and concentrate the liposomal API formulation to a final desired strength. In this case, the retentate contains the liposomal API formulation and the permeate acts as a waste stream. See, e.g., Figures 3-6 for Examples of processes that can be employed in the methods provided herein.

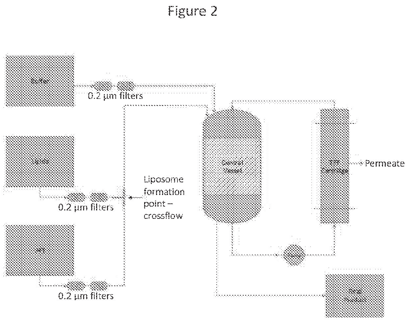

In embodiments provided herein, TFF for the buffer exchange and concentration in liposomal API formulation manufacturing is balanced to support continuous operation. A batch mode design for this operation entails a TFF step where the liposome-containing retentate is returned to the central vessel and the permeate/waste stream is made up with a feed of fresh buffer (constant-weight diafiltration), facilitating the buffer exchange. Once buffer exchange is complete, the product is concentrated to the desired strength by ceasing buffer addition (Figures 1, 2). In contrast, in particular embodiments provided herein, continuous buffer exchange and/or a concurrent concentration step are employed.

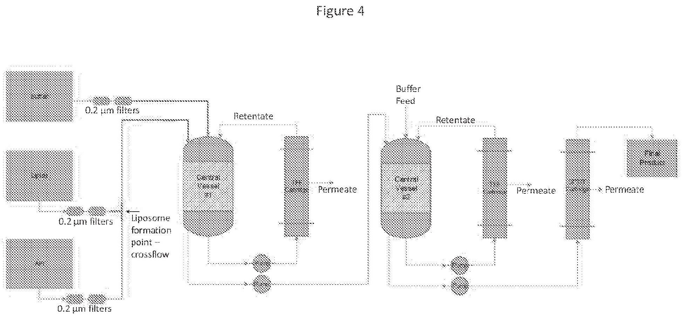

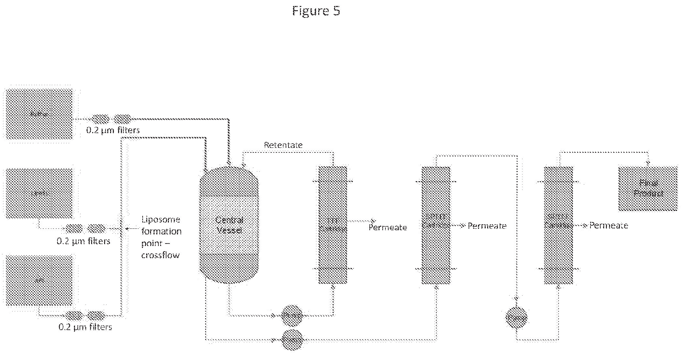

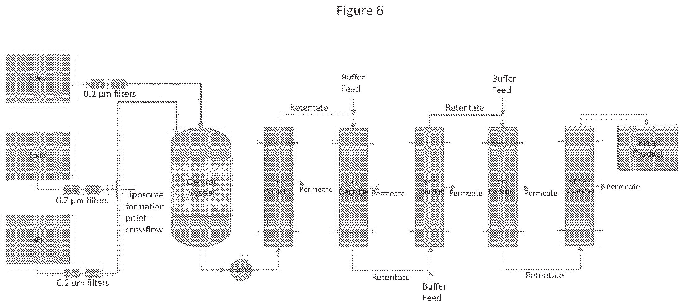

Depending on the composition of the incoming feeds and specification of the desired end formulation, various arrangements for a continuous operation can be employed. A single vessel buffer exchange TFF system with single stage concurrent concentrating SPTFF serves as one embodiment for a continuous design (Figure 3). If steady state diafiltration or single pass concentration are not able to achieve the required rate of buffer exchange or concentration with a single stage, additional stages may be added (Figures 4, 5). Additionally, more compact designs for continuous buffer exchange, such as the Cadence^{™} In-line Diafiltration Module (ILDF), are becoming available and can be employed in a continuous liposomal manufacturing process provided herein (*see, e.g.,* Gjoka et al. (2017) Platform for Integrated Continuous Bioprocessing. BioPharm International. 30:7, pp. 26-32, incorporated by reference herein in its entirety for all purposes). An ILDF design concluding with SPTFF, without wishing to be bound by theory, is thought to eliminate the need for multiple vessels to support continuous buffer exchange (Figure 6). Moreover, the ILDF design in Figure 6 can be modified, e.g., to include additional TFF units in series and/or parallel, for example, an additional, 1, 2, 3, 4, 5 or 6 TFF Units in series and/or parallel. Other ILDF system architectures amenable for use with the methods provided herein are found in U.S. Patent Application Publication No. 2017/0225123, the disclosure of which is incorporated by reference herein in its entirety for all purposes.

During manufacturing of liposomal formulations, there is allowable and expected variability in capture efficiency of the API. In a batch process, this is compensated for by offline in-process measurement of active ingredient concentration prior to the concentration step. Measurements such as flow rates, mass, and density provide a level of control that can be implemented in a continuous operation provided herein. In another embodiment, real-time concentration measurement such as in-line high performance liquid chromatography (HPLC) is employed. In another embodiment, rapid HPLC, which reduces off-line testing time from 60 minutes to 4 minutes is employed to measure concentration of liposomal API product during the manufacturing process (Kumar, V., Joshi, V., A Rapid HPLC Method for Enabling PAT Application for Processing of GCSF. LCGC North America. 2013, 31:11, 948-953, incorporated by reference herein in its entirety for all purposes). Other in-line measurements, such as particle size, in one embodiment, are employed. Particle size measurements, in one embodiment, are used to correlate size to concentration of the liposomal API product.

In one embodiment provided herein, the continuous manufacturing process is set up using pre-sterilized componentry and/or steam-in-place (SIP) equipment, and the feed solutions (API containing aqueous solution, lipid in organic solvent, or buffer) must enter the system through sterilizing filters containing a pore size of typically 0.2 µm or less. In one embodiment, the capability (ability of the filter to remove given concentrations of organism) and/or duration (time of use before grow-through of an organism compromises the filter) of the sterile filtration step is validated prior to implementing one or both in the continuous manufacturing methods provided herein. In one embodiment of the methods provided herein, a massively redundant filtration design or a sequential use of a parallel filtration pathways is employed. Without wishing to be bound by theory, it is thought that sequential use of parallel pathways is a viable solution since multiple redundant pathways can cause significant pressure drop issues.

In one embodiment, the API encapsulated by the liposomal manufacturing processes provided herein is an antiinfective. Antiinfectives are agents that act against infections, such as bacterial, mycobacterial, fungal, viral or protozoal infections. Antiinfectives that can be liposomally encapsulated by the methods provided herein include but are not limited to aminoglycosides (e.g., streptomycin, gentamicin, tobramycin, amikacin, netilmicin, kanamycin, and the like), tetracyclines (such as chlortetracycline, oxytetracycline, methacycline, doxycycline, minocycline and the like), sulfonamides (e.g., sulfanilamide, sulfadiazine, sulfamethaoxazole, sulfisoxazole, sulfacetamide, and the like), paraaminobenzoic acid, diaminopyrimidines (such as trimethoprim, often used in conjunction with sulfamethoxazole, pyrazinamide, and the like), quinolones (such as nalidixic acid, cinoxacin, ciprofloxacin and norfloxacin and the like), penicillins (such as penicillin G, penicillin V, ampicillin, amoxicillin, bacampicillin, carbenicillin, carbenicillin indanyl, ticarcillin, azlocillin, mezlocillin, piperacillin, and the like), penicillinase resistant penicillin (such as methicillin, oxacillin, cloxacillin, dicloxacillin, nafcillin and the like), first generation cephalosporins (such as cefadroxil, cephalexin, cephradine, cephalothin, cephapirin, cefazolin, and the like), second generation cephalosporins (such as cefaclor, cefamandole, cefonicid, cefoxitin, cefotetan, cefuroxime, cefuroxime axetil; cefmetazole, cefprozil, loracarbef, ceforanide, and the like), third generation ccphalosporins (such as cefepime, ccfopcrazonc, cefotaxime, ceftizoxime, ceftriaxone, ceftazidime, cefixime, cefpodoxime, ceftibuten, and the like), other beta-lactams (such as imipenem, meropenem, aztreonam, clavulanic acid, sulbactam, tazobactam, and the like), betalactamase inhibitors (such as clavulanic acid), chlorampheriicol, macrolides (such as erythromycin, azithromycin, clarithromycin, and the like), lincomycin, clindamycin, spectinomycin, polymyxin B, polymixins (such as polymyxin A, B, C, D, El(colistin A), or E2, colistin B or C, and the like) colistin, vancomycin, bacitracin, isoniazid, rifampin, ethambutol, ethionamide, aminosalicylic acid, cycloserine, capreomycin, sulfones (such as dapsone, sulfoxone sodium, and the like), clofazimine, thalidomide, or any other antibacterial agent that can be lipid encapsulated. Antiinfectives can include antifungal agents, including polyene antifungals (such as amphotericin B, nystatin, natamycin, and the like), flucytosine, imidazoles (such as n-ticonazole, clotrimazole, econazole, ketoconazole, and the like), triazoles (such as itraconazole, fluconazole, and the like), griseofulvin, terconazole, butoconazole ciclopirax, ciclopirox olamine, haloprogin, tolnaftate, naftifine, terbinafine, or any other antifungal that can be lipid encapsulated or complexed. Discussion and the examples are directed primarily toward amikacin but the scope of the application is not intended to be limited to this antiinfective. Combinations of APIs can be used.

In one embodiment, the API is an aminoglycoside, quinolone, a polyene antifungal or a polymyxins.

In one embodiment, the API is an aminoglycoside. In a further embodiment, the aminoglycoside is an aminoglycoside free base, or its salt, solvate, or other non-covalent derivative. In a further embodiment, the aminoglycoside is amikacin. Included as suitable aminoglycosides used in the API formulations of the present invention are pharmaceutically acceptable addition salts and complexes of APIs. In cases where the compounds may have one or more chiral centers, unless specified, the present invention comprises each unique racemic compound, as well as each unique nonracemic compound. In cases in which the active agents have unsaturated carbon-carbon double bonds, both the cis (Z) and trans (E) isomers are within the scope of this invention. In cases where the active agents exist in tautomeric forms, such as keto-enol tautomers, each tautomeric form is contemplated as being included within the invention. Amikacin, in one embodiment, is present in the pharmaceutical formulation as amikacin base, or amikacin salt, for example, amikacin sulfate or amikacin disulfate. In one embodiment, a combination of one or more of the above aminoglycosides is used in the formulations, systems and methods described herein. In a further embodiment, the combination comprises amikacin.

In one embodiment, the API is amikacin, or a pharmaceutically acceptable salt thereof. In a further embodiment, the amikacin is amikacin sulfate.

In yet another embodiment, the API is an aminoglycoside selected from amikacin, apramycin, arbekacin, astromicin, capreomycin, dibekacin, framycetin, gentamicin, hygromycin B, isepamicin, kanamycin, neomycin, netilmicin, paromomycin, rhodestreptomycin, ribostamycin, sisomicin, spectinomycin, streptomycin, tobramycin, verdamicin, or a combination thereof.

In yet another embodiment, the API is an aminoglycoside selected from AC4437, amikacin, apramycin, arbekacin, astromicin, bekanamycin, boholmycin, brulamycin, capreomycin, dibekacin, dactimicin, etimicin, framycetin, gentamicin, H107, hygromycin, hygromycin B, inosamycin, K-4619, isepamicin, KA-5685, kanamycin, neomycin, netilmicin, paromomycm, plazomicin, ribostamycin, sisomicm, thodestreptomycin, sorbistin, spectinomycin, sporaricin, streptomycin, tobramycin, verdamicin, vertilmicin, or a combination thereof.

In one embodiment, the API comprises a glycopeptide antibiotic. Glycopeptide antibiotics, including vancomycin and teicoplanin, are large, rigid molecules that inhibit a late stage in bacterial cell wall peptidoglycan synthesis. Glycopeptides are characterized by a multi-ring peptide core containing six peptide linkages, an unusual triphenyl ether moiety, and sugars attached at various sites. Over 30 antibiotics designated as belonging to the glycopeptide class have been reported. Among the glycopeptides, vancomycin and teicoplanin are used widely and are recommended for treatment of severe infections, especially those caused by multiple-drug-resistant Gram-positive pathogens. The glycopeptide avoparcin has been introduced as a growth promoter in animal husbandry in the past, and represents the main reservoir for the VanA type of vancomycin resistance in enterococci. Semisynthetic derivatives of vancomycin and teicoplanin, lipoglycopeptides, showed an extended spectrum of activity against multi-resistant and partly vancomycin-resistant bacteria (Reynolds (1989). Eur. J. Clin Microbiol Infect Dis 8, pp. 943-950; Nordmann et al. (2007). Curr. Opin. Microbiol. 10, pp. 436-440). Each of the publications referenced in this paragraph are incorporated by reference herein in their entireties.

Glycopeptide antibiotics are active against Gram-positive organisms and a few anaerobes. The main indications for glycopeptide antibiotics are infections caused by beta-lactamase-producing *Staphylococcus aureus* (for which beta-lactamase-resistant penicillins, cephalosporins, and combinations of penicillins with inhibitors of beta-lactamases proved safer alternatives), and colitis caused by *Colstridium difficile.* The emergence and rapid spread of methicillin-resistant *S*. *aureus* (MRSA) strains, which were resistant not only to all beta-lactams but also to the main antibiotic classes, renewed the interest in vancomycin and pushed teicophalnin, another natural glycopeptide, onto the market. Teicoplanin is comparable to vancomycin in terms of activity, but presents pharmacokinetic advantages, such as prolonged half-life, allowing for a once-daily administration (van Bambeke F., Curr. Opin. Pharm., 4(5):471-478).

A representative number of glycopeptides that can be used in the compositions of the present invention are provided in **Table 2.** The antibiotic complexes are listed in alphabetical order along with the structure type producing organism. These metabolites are elaborated by a diverse group of actinomycetes ranging from the more prevalent *Streptomyces* species to the relatively rare genera of *Streptosporangium* and *Saccharomonospora.* The less common *Actionplanes* and *Amycolatopsis* account for almost half of the producing organisms (Nagarajan, R., Glycopeptide Antibiotics, CRC Press, 1994, incorporated by reference herein in its entirety).

| **Table 2. Glycopeptide Antibiotics and Producing Organisms** | | |
|---|---|---|
| **Antibiotic** | **Type** | **Producing Organism** |
| A477 | ND | *Actinoplanes sp.* NRRL 3884 |
| A35512 | III | *Streptomyces candidus* NRRL 8156 |
| A40926 | IV | *Actinomadura sp.* ATTC39727 |
| A41030 | III | *Streptomyces virginiae* NRRL 15156 |
| A42867 | I | *Nocardia sp.* ATTC 53492 |
| A47934 | III | *Streptomyces toyocaensis* NRRL 15009 |
| A80407 | III | *Kibdelosporangium philippinensis* NRRL 18198 or NRRL 18199 |
| A82846 | I | *Amycolatopsis orientalis* NRRL 18100 |
| A83850 | I | *Amycolatopsis albus* NRRL 18522 |
| A84575 | I | *Streptosporangium cameum* NRRL 18437, 18505 |
| AB-65 | ND | *Saccharomonospora viride* T-80 FERM-P 2389 |
| Actaplanin | III | *Actinoplanes missouriensis* ATCC 23342 |
| Actinoidin | II | *Proactinomyces actinoides* |
| Ardacin | IV | *Kibdelosporangium aridum* ATCC 39323 |
| Avoparcin | II | *Streptomyces candidus* NRRL 3218 |
| Azureomycin | ND | *Pseudonocardia azurea* NRRL11412 |
| Chloroorienticin | I | *Amyclolatopsis orientalis* PA-45052 |
| Chloropolysporin | II | *Micropolyspora sp.* FERM BP-538 |
| Decaplanin | I | *Kibdelosporangium deccaensis* DSM 4763 |
| N-demethylvancomycin | I | *Amycolatopsis orientalis* NRRL 15252 |
| Eremomycin | I | *Actinomycetes sp.* INA 238 |
| Galacardin | II | *Actinomycetes strain* SANK 64289 FERM P-10940 |
| Helvecardin | II | *Pseudonocardia compacta* subsp. helvetica |
| Izupeptin | ND | *Norcardia* AM-5289 FERM P-8656 |
| Kibdelin | IV | *Kibdelosporangium aridum* ATCC 39922 |
| LL-AM374 | ND | *Streptomyces eburosporeus* NRRL 3582 |
| Mannopeptin | ND | *Streptomyces platenis* FS-351 |
| MM45289 | I | *Amycolatopsis orientalis* NCIB12531 |
| MM47761 | I | *Amycolatopsis orientalis* NCIB 12608 |
| MM47766 | II | *Amycolatopsis orientalis* NCBI 40011 |
| MM55266 | IV | *Amycolatopsis sp.* NCIB 40089 |
| MM55270 | ND | *Amycolatopsis sp.* NCIB 40086 |
| OA-7653 | I | *Streptomyces hygromscopicus* ATCC 31613 |
| Orienticin | I | *Nocardia orientalis* FERM BP-1230 |
| Parvodicin | IV | *Actinomadura parvosata* ATCC 532463 |
| Ristocetin | III | *Amycolatopsis orientalis* subsp. lurida NRRL 2430 |
| Ristomycin | III | *Proactinomyces fructifers* |
| Synmonicin | II | *Synnemomyces mamnoorii* ATCC 53296 |
| Teicoplanin | IV | *Actinoplanes teichomyceticus* ATCC 31121 |
| UK-68597 | III | *Actinoplanes* ATCC 53533 |
| UK-69542 | III | *Saccharothix aerocolonigenes* |
| UK-72051 | I | *Amycolatopsis orientalis* |
| Vancomycin | I | *Amycolaroposis orientalis* NRRL 2450 |

According to another embodiment, the glycopeptide antibiotic used in the composition of the present invention includes, but is not limited to, A477, A35512, A40926, A41030, A42867, A47934, A80407, A82846, A83850, A84575, AB-65, Actaplanin, Actinoidin, Ardacin, Avoparcin, Azureomycin, Chloroorienticin Chloropolysporin, Decaplanin, N-demethylvancomycin, Eremomycin, Galacardin, Helvecardin Izupeptin, Kibdelin, LL-AM374, Mannopeptin, MM45289, MM47761, MM47766, MM55266, MM55270, OA-7653, Orienticin, Parvodicin, Ristocetin, Ristomycin, Synmonicin, Teicoplanin, UK-68597, UK-69542, UK-72051, vancomycin, and a mixture thereof.

According to one embodiment, the API is vancomycin. Vancomycin is a water soluble amphoteric glycopeptide bactericidal antibiotic that inhibits gram-positive bacterial mucopeptide biosynthesis. It consists of a tricyclic nonribosomal heptapeptide core structure to which is attached a disaccharide unit consisting of the aminodeoxy sugar, vancosamine, and D-glucose. This natural antibiotic of ~1450 Daltons is obtained from *Streptomyces orientalis* (also known as; *Nocardia orientalis,* or *Amycolatopsis orientalis).* Vancomycin has one carboxyl group with pKa 2.18, and two amino groups: primary amine with pKa 7.75 and the secondary amine with pKa 8.89. At sub-physiological pH vancomycin has a net positive charge.

In another embodiment, the API is oritavancin (LY333328). Oritavancin is obtained by reductive alkylation with 4' chloro-biphenylcarboxaldehyde of the natural glycopeptide chloroeremomycin, which differs from vancomycin by the addition of a 4-epi-vancosamine sugar and the replacement of the vancosamine by a 4-epivancosamine (Cooper, R. et al., J Antibiot (Tokyo) 1996, 49:575-581, incorporated by reference herein in its entirety). Although oritavancin presents a general spectrum of activity comparable to that of vancomycin, it offers considerable advantages in terms of intrinsic activity (especially against streptococci), and remains insensitive to the resistance mechanisms developed by staphylococci and enterococci. Because the binding affinity of vancomycin and oritavancin to free D-Ala-D-Ala and D-Ala-D-Lac are of the same order of magnitude, the difference in their activity has been attributed to the cooperative interactions that can occur between the drug and both types of precursors in situ. The previous study suggested that the effect is caused possibly by a much stronger ability to dimerize and the anchoring in the cytosolic membrane of the chlorobiphenyl side chain (Allen, et al., FEMS Microbiol Rev, 2003, 26:511-532, incorporated by reference herein).

In another embodiment, the API is telavancin (TD-6424). Telavancin is a semi-synthetic derivative of vancomycin, possessing a hydrophobic side chain on the vancosamine sugar (decylaminoethyl) and a (phosphonomethyl) aminomethyl substituant on the cyclic peptidic core (van Bambeke, F., Curr. Opin. Pharm., 4(5): 471-478; Judice, J et al., Bioorg Med Chem Lett 2003, 13: 4165-4168, incorporated by reference herein in its entirety). The length of the hydrophobic side chain was chosen to reach a compromise between optimized activity against MRSA (8-10 carbons) and VanA enterococci (12-16 carbons). Pharmacological studies suggest that the enhanced activity of telavancin on *S. pneumoniae, S. aureus* (to a lesser extent), and staphylococci or enterococci harboring the vanA gene cluster results from a complex mechanism of action which, on the basis of data obtained with close analogs, involves a perturbation of lipid synthesis and possibly membrane disruption.

In even another embodiment, the API is dalbavancin (BI 397). Dalbavancin is a semi-synthetic derivative of A40926, a glycopeptide with a structure related to that of teicoplanin. As with oritavancin and telavancin, dalbavancin is more active against *S. pneumoniae* than are conventional glycopeptides, and its activity against *S. aureus* is also substantially improved, which was not observed with the semi-synthetic derivatives of vancomycin. However, studies have shown that it is not more active than teicoplanin against enterococci harboring the VanA phenotype of resistance to glycopeptides.

The lipid component used in the continuous manufacturing process described herein in one embodiment, comprises a net neutral lipid, or a combination of net neutral lipids. In one embodiment, the lipid component is free of anionic lipids. In one embodiment, the lipid is a phospholipid, including but not limited to, a phosphatidylcholine such as dipalmitoylphosphatidylcholine or dioleoylphosphatidylcholine; a sterol, including, but not limited to, cholesterol; or a combination of a phosphatidylcholine and a sterol (e.g., cholesterol).

Examples of the lipid component that can be used in preparing the stabilized lipid-based glycopeptide antibiotic composition of the present invention includes, but is limited to, phosphatidylcholine (PC), phosphatidylglycerol (PG), phosphatidylinositol (PI), phosphatidylserine (PS), phosphatidylethanolamine (PE), phosphatidic acid (PA), egg phosphatidylcholine (EPC), egg phosphatidylglycerol (EPG), egg phosphatidylinositol (EPI), egg phosphatidylserine (EPS), phosphatidylethanolamine (EPE), phosphatidic acid (EPA), soy phosphatidylcholine (SPC), soy phosphatidylglycerol (SPG), soy phosphatidylserine (SPS), soy phosphatidylinositol (SPI), soy phosphatidylethanolamine (SPE), soy phosphatidic acid (SPA), hydrogenated egg phosphatidylcholine (HEPC), hydrogenated egg phosphatidylglycerol (HEPG), hydrogenated egg phosphatidylinositol (HEPI), hydrogenated egg phosphatidylserine (HEPS), hydrogenated phosphatidylethanolamine (HEPE), hydrogenated phosphatidic acid (HEPA), hydrogenated soy phosphatidylcholinc (HSPC), hydrogenated soy phosphatidylglycerol (HSPG), hydrogenated soy phosphatidylserine (HSPS), hydrogenated soy phosphatidylinositol (HSPI), hydrogenated soy phosphatidylethanolamine (HSPE), hydrogenated soy phosphatidic acid (HSPA), dipalmitoylphosphatidylcholine (DPPC), dimyristoylphosphatidylcholine (DMPC), dimyristoylphosphatidylglycerol (DMPG), dipalmitoylphosphatidylglycerol (DPPG), distearoylphosphatidylcholine (DSPC), distearoylphosphatidylglycerol (DSPG), dioleoylphosphatidylcholine (DOPC), dioleylphosphatidylethanolamine (DOPE), palmitoylstearoylphosphatidyl-choline (PSPC), palmitoylstearolphosphatidylglycerol (PSPG), mono-oleoyl-phosphatidylethanolamine (MOPE), tocopherol, tocopherol hemisuccinate, cholesterol sulfate, cholesteryl hemisuccinate, cholesterol derivatives, ammonium salts of fatty acids, ammonium salts of phospholipids, ammonium salts of glycerides, myristylamine, palmitylamine, laurylamine, stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2. 3-di-(9-(Z)-octadecenyloxy)-prop-1-yl-N,N,N-trimethylammonium chloride (DOTMA), 1, 2-bis(oleoyloxy)-3-(trimethylammonio)propane (DOTAP), distearoylphosphatidylglycerol (DSPG), dimyristoylphosphatidylacid (DMPA), dipalmitoylphosphatidylacid (DPPA), distearoylphosphatidylacid (DSPA), dimyristoylphosphatidylinositol (DMPI), dipalmitoylphosphatidylinositol (DPPT), distearoylphospatidylinositol (DSPI), dimyristoylphosphatidylserine (DMPS), dipalmitoylphosphatidylserine (DPPS), distearoylphosphatidylserine (DSPS), or a mixture thereof.

In another embodiment, the lipid component used in the continuous manufacturing process of the present invention comprises palmitoylstearoylphosphatidylcholine (PSPC), palmitoylstearoylphosphatidylglycerol (PSPG), triacylglycerol, diacylglycerol, seranide, sphingosine, sphingomyelin, a single acylated phospholipid, such as mono-oleoyl-phosphatidylethanol amine (MOPE), or a combination thereof.

In another embodiment, the lipid component used in the continuous manufacturing process comprises an ammonium salt of a fatty acid, a phospholipid, sterol, a phosphatidylglycerols (PG), a phosphatidic acid (PA), a phosphotidylcholine (PC), phosphatidylinositol (PI) or a phosphatidylserine (PS). The fatty acid can be a fatty acids of carbon chain lengths of 12 to 26 carbon atoms that is either saturated or unsaturated. Some specific examples include, but are not limited to, myristylamine, palmitylamine, laurylamine and stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2, 3- di-(9 (Z)-octadecenyloxy)-prop-1-yl-N,N,N-trimethylammonium chloride (DOTMA) and 1, 2-bis(oleoyloxy)-3-(trimethylammonio)propane (DOTAP).

According to another embodiment, the lipid component comprises a phosphatidylcholine. In a further embodiment, the phosphatidylcholine is dipalmitoylphosphatidylcholine (DPPC) or palmitoyloleoylphosphatidylcholine (POPC). In even a further embodiment, the phosphatidylcholine comprises DPPC.

According to another embodiment, the lipid component comprises a phosphatidylglycerol. In a further embodiment, the phosphatidylglycerol is 1-palmitoyl-2-oleoyl-sn-glycero-3-phosphoglycerol (POPG).

According to another embodiment, the lipid component comprises a sterol, including, but not limited to, cholesterol and ergosterol. In one embodiment, the lipid component comprises a phospholipid and a sterol. In a further embodiment, the sterol is cholesterol.

The lipid-to-API weight ratio of the liposomal encapsulated API provided herein, in one embodiment, is 3 to 1 or less, 2.5 to 1 or less, 2 to 1 or less, 1.5 to 1 or less, or 1 to 1 or less. The lipid to API ratio of the liposomal encapsulated API provided herein, in another embodiment, is less than 3 to 1, less than 2.5 to 1, less than 2 to 1, less than 1.5 to 1, or less than 1 to 1. In a further embodiment, the lipid to API ratio is about 0.7 to 1 or less or about 0.7 to 1. In even a further embodiment, the API is an aminoglycoside, e.g., amikacin or a pharmaceutically acceptable salt thereof.

The lipid-to-API weight ratio (lipid:API) of the liposomal encapsulated API provided herein, in one embodiment, is from about 3:1 to about 0.5:1, from about 2.5:1 to about 0.5:1, from about 2:1 to about 0.5:1, from about 1.5:1 to about 0.5:1, or from about 1:1 to about 0.5:1. In a further embodiment, the API is an aminoglycoside, e.g., amikacin or a pharmaceutically acceptable salt thereof.

### EXAMPLES

The present invention is further illustrated by reference to the following Examples. However, it should be noted that the Examples, like the embodiments described above, are illustrative and not to be construed as restricting the scope of the invention in any way.

### Example 1 - Case Study of Batch and Continuous Liposome Manufacturing: Processes

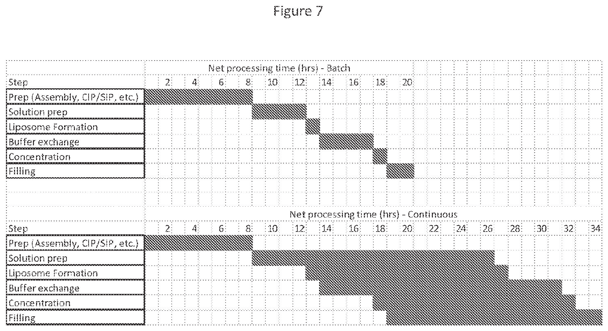

For the purposes of the case study, the following options are compared; (1) a batch process design producing 2500 filled units from a 1 hr. liposome formation step with supporting batch process steps and (2) a continuous process design allowing for a 24 hr. liposome formation step with concurrent continuous unit operations. The batch process is based on a process used for early phase clinical production. It is assumed that the batch and continuous designs are using similar scale equipment with similar processing rates. A summary of the unit operations and processing times is in Figure 7.

The batch process is able to produce 2500 filled units in 20 hr. of total processing time including preparation (assembly, CIP/SIP, etc.). This calculates to 125 units/hr. The continuous process with a 24 hr. liposome formation step produces 18,750 filled units in 34 hr. of total process time or 551 units/hr. This translates to a 4.4-fold increase in output for the same overhead costs and a 7.5-fold output increase for the same process preparation costs and single-use componentry costs (sterilizing filters, TFF cartridges). This ignores the additional capital expenses needed to achieve one of the continuous designs previously mentioned (e.g., set forth at Figures 3-6).

Another way to compare the processes is by their ability to fulfill a given production forecast. For a forecast of 1 million units per year, the continuous design requires the 34-hr. process to be run approximately once per week. For the batch design, the 20-hr. process would have to be run more than once per day, necessitating multiple lines running at a higher rate to fulfill the forecast.

By converting the early phase clinical scale production line to a continuous operation, not only are cost savings and higher throughput achieved, but the need for scaling up the process is alleviated, which eliminates the need for supporting process development work and large-scale capital equipment purchases.

### Example 2 -Continuous Liposome Manufacturing

This example outlines a continuous inline dial filtration (ILDF)/concentration of a liposomal amikacin formulation having a lipid component consisting of DPPC and cholesterol. This Example is concerned with understanding the operating conditions/parameters for the continuous in-line diafiltration module.

### Equipment and Components

The equipment and components in Table 3 below was used for both experiments executed under this Example. The ILDF setup utilized two standard peristaltic pumps for operations - the first to control the feed and retentate, and the second to control the buffer injection. The ILDF included six fluid treatment modules. A fluid treatment module comprises a filtration membrane, feed channel and permeate channel (i.e., the diagram shown in Figure 6 with one additional fluid treatment module).

| **Table 3** | | |
|---|---|---|
| **Equipment** | **Manufacturer** | **Vendor Part No.** |
| Cadence Inline Diafiltration Module | Pall Corporation | DFOS030T120612 |
| 135L SS Jacketed Vessel | Sharpsville | 1103 |
| 100L SS Jacketed Vessel | Lee Industries | B8783-A |
| Tubing Flowpaths with Sensors | Pall Corporation | DFOS030T120612 |
| 1000L PVDF Vessel | Terracon | Custom |
| Infusion Peristaltic Pumps | Watson Marlow | 520U |
| Masterflex L/S Pumps for Saline (DF control) and Feed/Retentate | Cole-Parmer | EW-07522-20 |
| Masterflex Easy Load II Pump Heads | Cole-Parmer | EW-77201-60 |
| Masterflex L/S cartridge pump head (6 channel, 6 roller) | Cole-Parmer | EW-07519-15 |
| Masterflex L/S pump head cartridges | Cole-Parmer | EW-07519-75 |
| Pressure Monitor (Feed, Retentate) | PendoTECH | PMAT4A-BAR |
| Balance for Raw Material Weighing | Sartorius | Signum 1 |
| Flow Meters | Endress Hauser | 83P08 |

### Solution Preparation

Prior to beginning each experiment, all product contact surfaces in the process train were either cleaned using 0.1N NaOH or replaced with new components where appropriate. Post cleaning rinsing with RODI water was completed until neutral pH was achieved. Following cleaning, the amikacin solution and saline was prepared, followed by lipid solution. All raw materials weighed were within expected accuracy from the target. All raw materials weights and additional processing information related to infusion, diafiltration and concentration was recorded during processing. Raw materials for solution preparation are provided in Table 4 below.

| **Table 4.** | | |
|---|---|---|
| **Solution** | **Raw Materials** | **Vendor** |
| Amikacin | Water | RODI |
| | Amikacin | ACS Dobfar |
| | NaOH | J.T. Baker |
| Lipid | Ethanol | PharmcoAaper |
| | Cholesterol | Dishman |
| | DPPC | Lipoid |
| Saline | Water | RODI |
| | NaCl | J.T. Baker |

### Amikacin-Lipid Infusion

During processing, the Melfi system records flow rates, pressure, temperature, vessel weight and time. Amikacin and lipid infusion was carried out via an in-line method to create 2L of a liposomal amikacin suspension, as described in U.S. Patent No. 7,718,189, the disclosure of which is incorporated by reference herein in its entirety.

### Inline Diafiltration

The 2L of infused material was collected under the skid and processed by ILDF. PendoTECH's custom data acquisition software was used to record and log all process data for the duration of the diafiltration. After ~200mL of product was diafiltered at one set of flowrates (Trial 1), the pump settings were changed and ~200mL of product was collected at another set of flowrates (Trial 2). See Table 5 for a summary of process data collected throughout the experiments.

| **Table 5. Process data summary.** | | | | | | | | |
|---|---|---|---|---|---|---|---|---|
| **Sample** | **Infuslon Flow rate (mL/min)** | **Buffer Flow rate (mL/min)** | **Average Feed Pressure (psi)** | **Average TMP (psi)** | **Average Feed Conductivity (mS)** | **Average Recentate Conductivity (mS)** | **Avg. Feed Temp (C)** | **Avg. Retentate Temp (C)** |
| 1 | 10 | 30 | 25.0 | 0.88 | 6.89 | 454 | 25.3 | 23.6 |
| 2 | 5 | 25 | 24.4 | 0.87 | 6.83 | 364 | 24.7 | 23.7 |

### Analytical Results

Table 6 provides the initial analytical results from the experiments.

| **Table 6. Analytical Results Summary** | | | | | | |
|---|---|---|---|---|---|---|
| **Sample** | **Infusion Flowrate** | **Buffer Flowrate** | **Amikacin Conc.** | **Cholesterol Conc.** | **DPPC Conc.** | **Lipid-to-API weight ratio** |
| 1 | 10 mL/min | 30 mL/min | 6 mg/mL | 2 mg/mL | 3 mg/mL | 0.83 |
| 2 | 5 mL/min | 25 mL/min | 14 mg/mL | 4 mg/mL | 8 mg/mL | 0.86 |

All publications, protocols, patents and patent applications cited herein are incorporated herein by reference in their entireties for all purposes.

While the described invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the invention. All such modifications are intended to be within the scope of the claims appended hereto.

### Embodiments

Various embodiments of the invention are defined in the clauses below:
1. A method for making a liposomal API formulation, comprising,
   mixing a lipid solution comprising a lipid dissolved in an organic solvent with an aqueous API solution, wherein the lipid solution and aqueous API solution are mixed from two separate streams in an in-line fashion, and wherein a liposomal encapsulated API is formed at the intersection of the two streams,
   introducing the liposomal encapsulated API into a first central vessel comprising a first inlet, a second inlet, a first outlet and a second outlet, through the first inlet, wherein the first outlet is in fluid communication with an inlet of a first tangential flow filtration (TFF) unit comprising the inlet and a first and second outlet, wherein the first outlet of the first TFF unit is in fluid communication with the second inlet of the first central vessel and the second outlet of the first TFF unit is a waste (permeate) outlet; and the second outlet of the first central vessel is in fluid communication with an inlet of a second TFF unit comprising the inlet and a first and second outlet, wherein the first outlet of the second TFF unit is a retentate outlet and the second outlet of the second TFF unit is a waste (permeate) outlet;
   continuously flowing the liposomal encapsulated API into the first TFF unit for a first period of time, wherein the liposomal encapsulated API enters the first TFF unit through the TFF inlet and exits through the first outlet;
   flowing the liposomal encapsulated API from the first central vessel through the inlet of the second TFF unit for a second period of time; and
   collecting the liposomal API formulation from the first outlet of the second TFF unit.
2. A method for making a liposomal API formulation, comprising,
   mixing a lipid solution comprising a lipid dissolved in an organic solvent with an aqueous API solution, wherein the lipid solution and aqueous API solution are mixed from two separate streams in an in-line fashion, and wherein a liposomal encapsulated API is formed at the intersection of the two streams,
   introducing the liposomal encapsulated API into a first central vessel comprising an inlet and an outlet, through the inlet, wherein the outlet is in fluid communication with an inlet of a first tangential flow filtration (TFF) unit comprising the inlet and a first and second outlet, wherein the first outlet of the first TFF unit is in fluid communication with the inlet of a second TFF comprising the inlet and a first and second outlet, and the second outlet of the first TFF unit is a waste (permeate) outlet; and wherein the first outlet of the second TFF unit is a retentate outlet and the second outlet of the second TFF unit is a waste (permeate) outlet;
   flowing the liposomal encapsulated API into the first TFF unit for a first period of time, wherein the liposomal encapsulated API enters the first TFF unit through the TFF inlet and exits through the first outlet;
   flowing the liposomal encapsulated API from the first outlet of the first TFF through the inlet of the second TFF unit for a second period of time; and
   collecting the liposomal API formulation from the first outlet of the second TFF unit.
3. A method for making a liposomal API formulation, comprising,
   mixing a lipid solution comprising a lipid dissolved in an organic solvent with an aqueous API solution, wherein the lipid solution and aqueous API solution are mixed from two separate streams in an in-line fashion, and wherein liposomal encapsulated API is formed at the intersection of the two streams,
   introducing the liposomal encapsulated API into a first central vessel comprising a first inlet, a second inlet, a first outlet and a second outlet, through the first inlet, wherein the first outlet is in fluid communication with an inlet of a first tangential flow filtration (TFF) unit comprising the inlet and a first and second outlet, wherein the first outlet of the first TFF unit is in fluid communication with the second inlet of the first central vessel and the second outlet of the first TFF unit is a waste outlet; and the second outlet of the first central vessel is in fluid communication with a first inlet of a second central vessel,
   wherein the second central vessel comprises the first inlet, a second inlet, a first outlet and a second outlet, and the first outlet of the second central vessel is in fluid communication with an inlet of a second tangential flow filtration (TFF) unit comprising the inlet and a first and second outlet,
   wherein the first outlet of the second TFF unit is in fluid communication with the second inlet of the second central vessel, the second outlet of the second TFF unit is a waste outlet; the second outlet of the second central vessel is in fluid communication with an inlet of a third TFF unit comprising the inlet and a first and second outlet, wherein the first outlet of the third TFF unit is a retentate outlet and the second outlet of the third TFF unit is a waste (permeate) outlet;
   continuously flowing the liposomal encapsulated API into the first TFF unit for a first period of time, wherein the liposomal encapsulated API enters the first TFF unit through the TFF inlet and exits through the first outlet;
   flowing the liposomal encapsulated API from the first central vessel into the second central vessel for a second period of time;
   continuously flowing the liposomal encapsulated API into the second TFF unit from the second central vessel for a third period of time, wherein the liposomal encapsulated API enters the second TFF unit through the TFF inlet and exits through the first outlet;
   flowing the liposomal encapsulated API from the second central vessel through the inlet of the third TFF unit for a fourth period of time; and
   collecting the liposomal encapsulated API formulation from the first outlet of the third TFF unit.
4. The method of any one of clauses 1-3, wherein the mixing results in the formation of an API coacervate.
5. The method of clause 4, wherein the API coacervate initiates lipid bilayer formation around the API coacervate.
6. The method of any one of clauses 1-5, wherein the concentration of the API inside the liposome is higher than the external API concentration.
7. The method of any one of clauses 1-6, wherein a buffer is introduced into the first central vessel through a third inlet prior to the first period of time or during the first period of time.
8. The method of any one of clauses 1, 2 and 4-7, wherein the second TFF unit is a single pass TFF unit (SPTFF).
9. The method of any one of clauses 3-7, wherein the third TFF unit is a single pass TFF unit (SPTFF).
10. The method of any one of clauses 1, 2 and 4-8, further comprising flowing the liposomal encapsulated API from the first central vessel into one or more additional TFF units, prior to flowing the liposomal API formulation from the one or more additional TFF units into the second TFF unit, and collecting the liposomal API formulation from the first outlet of the second TFF unit.
11. The method of any one of clauses 3-9, further comprising flowing the liposomal encapsulated API from the second central vessel into one or more additional TFF units, prior to flowing the liposomal API formulation from the one or more additional TFF units into the third TFF unit, and collecting the liposomal API formulation from the first outlet of the third TFF unit.
12. The method of clause 10 or 11, wherein the one or more additional TFF units comprises one or more SPTFF units.
13. The method of any one of clauses 10-12, wherein the one or more additional TFF units comprise two or more TFF units connected in series.
14. The method of any one of clauses 10-12, wherein the one or more additional TFF units comprise two or more TFF units connected in parallel.
15. The method of any one of clauses 10-13, wherein the one or more additional TFF units comprises four TFF units connected in series.
16. The method of any one of clauses 10-12, wherein the one or more additional TFF units comprises a single TFF unit.
17. The method of any one of clauses 6-16, wherein the buffer is a sodium chloride buffer.
18. The method of any one of clauses 1-17, wherein the lipid comprises a phospholipid.
19. The method of any one of clauses 1-18, wherein the lipid comprises a phosphatidylcholine (PC), phosphatidylglycerol (PG), phosphatidylinositol (PI), phosphatidylserine (PS), phosphatidylethanolamine (PE) or phosphatidic acid (PA).
20. The method of clause 18, wherein the phospholipid is a phosphatidylcholine.
21. The method of any one of clauses 1-20, wherein the lipid comprises one or more net neutral lipids.
22. The method of clause 20, wherein the phosphatidylcholine is dipalmitoyl phosphatidylcholine (DPPC).
23. The method of any one of clauses 1-22, wherein the lipid comprises cholesterol.
24. The method of any one of clauses 1-17, wherein the lipid comprises egg phosphatidylcholine (EPC), egg phosphatidylglycerol (EPG), egg phosphatidylinositol (EPI), egg phosphatidylserine (EPS), phosphatidylethanolamine (EPE), phosphatidic acid (EPA), soy phosphatidylcholine (SPC), soy phosphatidylglycerol (SPG), soy phosphatidylserine (SPS), soy phosphatidylinositol (SPI), soy phosphatidylethanolamine (SPE), soy phosphatidic acid (SPA), hydrogenated egg phosphatidylcholine (HEPC), hydrogenated egg phosphatidylglycerol (HEPG), hydrogenated egg phosphatidylinositol (HEPI), hydrogenated egg phosphatidylserine (HEPS), hydrogenated phosphatidylethanolamine (HEPE), hydrogenated phosphatidic acid (HEPA), hydrogenated soy phosphatidylcholine (HSPC), hydrogenated soy phosphatidylglycerol (HSPG), hydrogenated soy phosphatidylserine (HSPS), hydrogenated soy phosphatidylinositol (HSPI), hydrogenated soy phosphatidylethanolamine (HSPE), hydrogenated soy phosphatidic acid (HSPA), dipalmitoylphosphatidylcholine (DPPC), dimyristoylphosphatidylcholine (DMPC), dimyristoylphosphatidylglycerol (DMPG), dipalmitoylphosphatidylglycerol (DPPG), distearoylphosphatidylcholine (DSPC), distearoylphosphatidylcholine (DSPG), dioleoylphosphatidylcholine (DOPC), dioleylphosphatidylethanolamine (DOPE), palmitoylstearoylphosphatidyl-choline (PSPC), palmitoylstearolphosphatidylglycerol (PSPG), mono-oleoyl-phosphatidylethanolamine (MOPE), tocopherol, tocopherol hemisuccinate, cholesterol sulfate, cholesteryl hemisuccinate, cholesterol derivatives, ammonium salts of fatty acids, ammonium salts of phospholipids, ammonium salts of glycerides, myristylamine, palmitylamine, laurylamine, stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2, 3-di-(9-(Z)-octadecenyloxy)-prop-1-yl-N,N,N-trimethylammonium chloride (DOTMA), 1, 2-bis(oleoyloxy)-3-(trimethylammonio)propane (DOTAP), distearoylphosphatidylglycerol (DSPG), dimyristoylphosphatidylacid (DMPA), dipalmitoylphosphatidylacid (DPPA), distearoylphosphatidylacid (DSPA), dimyristoylphosphatidylinositol (DMPI), dipalmitoylphosphatidylinositol (DPPI), distearoylphospatidylinositol (DSPI), dimyristoylphosphatidylserine (DMPS), dipalmitoylphosphatidylserine (DPPS), distearoylphosphatidylserine (DSPS), or a mixture thereof.
25. The method of any one of clauses 1-24, wherein the lipid comprises palmitoylstearoylphosphatidylcholine (PSPC), palmitoylstearoylphosphatidylglycerol (PSPG), triacylglycerol, diacylglycerol, seranide, sphingosine, sphingomyelin, or a single acylated phospholipid.
26. The method of clause 25, wherein the lipid comprises a single acylated phospholipid.
27. The method of clause 26, wherein the single acylated phospholipid is mono-oleoyl-phosphatidylethanol amine (MOPE).
28. The method of any one of clauses 1-17, wherein the lipid comprises myristylamine, palmitylamine, laurylamine and stearylamine, dilauroyl ethylphosphocholine (DEEP), dimyristoyl ethylphosphocholine (DMEP), dipalmitoyl ethylphosphocholine (DPEP) and distearoyl ethylphosphocholine (DSEP), N-(2, 3- di-(9 (Z)-octadecenyloxy)-prop-1-yl-N,N,N-trimethylammonium chloride (DOTMA), 1, 2-bis(oleoyloxy)-3-(trimethylammonio)propane (DOTAP), or a combination thereof.
29. The method of any one of clauses 1-17, wherein the lipid comprises DPPC and cholesterol.
30. The method of any one of clauses 1-17, wherein the lipid consists of DPPC and cholesterol.
31. The method of any one of clauses 1-29, wherein the lipid comprises palmitoyloleoylphosphatidylcholine (POPC).
32. The method of any one of clauses 1-29, wherein the lipid comprises 2-oleoyl-sn-glycero-3-phosphoglycerol (POPG).
33. The method of any one of clauses 1-32, wherein the API is an antiinfective.
34. The method of clause 33, wherein the antiinfective is an aminoglycoside, or a pharmaceutically acceptable salt thereof.
35. The method of clause 34, wherein the aminoglycoside is amikacin, or a pharmaceutically acceptable salt thereof.
36. The method of clause 35, wherein the amikacin is amikacin sulfate.
37. The method of clause 33, wherein the antiinfective is a tetracycline.
38. The method of clause 37, wherein the tetracycline is chlortetracycline, oxytetracycline, methacycline, doxycycline or minocycline.
39. The method of clause 33, wherein the antiinfective is a sulfonamide.
40. The method of clause 39, wherein the sulfonamide is sulfanilamide, sulfadiazine, sulfamethaoxazole, sulfisoxazole or sulfacetamide.
41. The method of clause 33, wherein the antiinfective is a quinolone.
42. The method of clause 41, wherein the quinolone is nalidixic acid, cinoxacin, ciprofloxacin or norfloxacin.
43. The method of clause 42, wherein the quinolone is ciprofloxaicin.
44. The method of clause 33, wherein the antiinfective a glycopeptide.
45. The method of clause 44, wherein the glycopeptide is a glycopeptide recited in Table 2.
46. The method of clause 45, wherein the glycopeptide is vancomycin, or a derivative thereof.
47. The method of any one of clauses 1-32, wherein the API is an antifungal drag.
48. The method of clause 47, wherein the antifungal drug is a polyene antifungal, flucytosine, an imidazole, a triazole, griseofulvin, terconazole, butoconazole ciclopirax, ciclopirox olamine, haloprogin, tolnaftate, naftifine or terbinafine.
49. The method of clause 34, wherein the aminoglycoside is amikacin, apramycin, arbekacin, astromicin, capreomycin, dibekacin, framycetin, gentamicin, hygromycin B, isepamicin, kanamycin, neomycin, netilmicin, paromomycin, rhodestreptomycin, ribostamycin, sisomicin, spectinomycin, streptomycin, tobramycin, verdamicin, a pharmaceutically acceptable salt thereof, or a combination thereof.
50. The method of clause 34, wherein the aminoglycoside is AC4437, amikacin, apramycin, arbekacin, astromicin, bekanamycin, boholmycin, brulamycin, capreomycin, dibekacin, dactimicin, etimicin, framycetin, gentamicin, H107, hygromycin, hygromycin B, inosamycin, K-4619, isepamicin, KA-5685, kanamycin, neomycin, netilmicin, paromomycm, plazomicin, ribostamycin, sisomicin, rhodestreptomycin, sorbistin, spectinomycin, sporaricin, streptomycin, tobramycin, verdamicin, vertilmicin, a pharmaceutically acceptable salt thereof, or a combination thereof.
51. The method of clause 47, wherein the antifungal drug is a polyene antifungal.
52. The method of clause 51, wherein the polyene antiifungal is amphotericin B, nystatin or natamycin.
53. The method of clause 47, wherein the antifungal drug is an imidazole.
54. The method of clause 53, wherein the imidazole is n-ticonazole, clotrimazole, econazole or ketoconazole.
55. The method of clause 47, wherein the antifungal drug is a triazole.
56. The method of clause 55, wherein the triazole is itraconazole or fluconazole.
57. The method of any one of clauses 1-56, wherein the lipid-to-API weight ratio of the collected liposomal API formulation is 3 to 1 or less, 2.5 to 1 or less, 2 to 1 or less, 1 .5 to 1 or less, or 1 to 1 or less.
58. The method of any one of clauses 1-56, wherein the lipid-to-API weight ratio of the collected liposomal API formulation is less than 3 to 1, less than 2.5 to 1, less than 2 to 1, less than 1.5 to 1, or less than 1 to 1.
59. The method of any one of clauses 1-56, wherein the lipid-to-API weight ratio of the collected liposomal API formulation is about 0.7 to 1.
60. The method of any one of clauses 1-56, wherein the lipid-to-API weight ratio of the collected liposomal API formulation is from about 3:1 to about 0.5:1, from about 2.5:1 to about 0.5:1, from about 2:1 to about 0.5:1, from about 1.5:1 to about 0.5:1, or from about 1:1 to about 0.5:1.
61. The method of any one of clauses 1-56, wherein the lipid-to-API weight ratio of the collected liposomal API formulation is from about 3:1 to about 0.5:1.
62. The method of any one of clauses 1-56, wherein the lipid-to-API weight ratio of the collected liposomal API formulation is from about 2.5:1 to about 0.5:1.
63. The method of any one of clause s 1-56, wherein the lipid-to-API weight ratio of the collected liposomal API formulation is from about 2:1 to about 0.5:1.
64. The method of any one of clauses 1-56, wherein the lipid-to-API weight ratio of the collected liposomal API formulation is from about 1:1 to about 0.5:1.

## Claims

1. A method for continuously manufacturing a liposomal active pharmaceutical ingredient (API) formulation, comprising,
continuously mixing a lipid solution comprising a lipid dissolved in an organic solvent with an aqueous API solution, wherein the lipid solution and aqueous API solution are mixed from two separate streams in an in-line fashion, wherein the mixing is not carried out in a microfluidic channel, and wherein a liposomal encapsulated API is formed continuously at the intersection of the two streams,
introducing the liposomal encapsulated API into a first central vessel comprising an inlet and an outlet, through the inlet, wherein the outlet is in fluid communication with an inlet of a first tangential flow filtration (TFF) unit comprising the inlet and a first and second outlet, wherein the first outlet of the first TFF unit is in fluid communication with an inlet of a second TFF unit comprising the inlet and a first and second outlet, and the second outlet of the first TFF unit is a waste (permeate) outlet; and wherein the first outlet of the second TFF unit is a retentate outlet and the second outlet of the second TFF unit is a waste (permeate) outlet;
continuously flowing the liposomal encapsulated API into the first TFF unit for a first period of time, wherein the liposomal encapsulated API enters the first TFF unit through the inlet of the first TFF unit and exits through the first outlet of the first TFF unit;
flowing the liposomal encapsulated API from the first outlet of the first TFF unit through the inlet of the second TFF unit for a second period of time; and
collecting the liposomal API formulation from the first outlet of the second TFF unit, wherein the liposomal encapsulated API is subjected to continuous in-line diafiltration via TFF prior to collecting the liposomal API formulation.

2. The method of claim 1, wherein the mixing results in the formation of an API coacervate.

3. The method of claim 1 or 2, wherein a buffer is introduced into the first central vessel through a third inlet prior to the first period of time or during the first period of time.

4. The method of any one of claims 1-3, further comprising flowing the liposomal encapsulated API from the first central vessel into one or more additional TFF units, prior to flowing the liposomal API formulation from the one or more additional TFF units into the second TFF unit, and collecting the liposomal API formulation from the first outlet of the second TFF unit.

5. The method of claim 3, wherein the buffer is a sodium chloride buffer.

6. The method of any one of claims 1-5, wherein the second TFF unit is a single pass TFF unit (SPTFF).

7. The method of any one of claims 1-6, wherein the lipid comprises (a) a phospholipid, (b) cholesterol, or both (a) and (b).

8. The method of claim 7, wherein the phospholipid is a phosphatidylcholine.

9. The method of claim 8, wherein the phosphatidylcholine is dipalmitoyl phosphatidylcholine (DPPC).

10. The method of any one of claims 1-6, wherein the lipid consists of DPPC and cholesterol.

11. The method of any one of claims 1-10, wherein the API is an antiinfective.

12. The method of claim 11, wherein the antiinfective is an aminoglycoside, or a pharmaceutically acceptable salt thereof.

13. The method of claim 12, wherein the aminoglycoside is amikacin, or a pharmaceutically acceptable salt thereof.

14. The method of claim 13, wherein the amikacin or pharmaceutically acceptable salt thereof is amikacin sulfate.

15. The method of any one of claims 1-14, wherein the lipid-to-API weight ratio of the collected liposomal API formulation is from 3:1 to 0.5:1, from 2.5:1 to 0.5:1, from 2:1 to 0.5:1, from 1.5:1 to 0.5:1, from 1:1 to 0.5:1, or 0.7 to 1.
